(19) Europäisches Patentamt
European Patent Office
Office européen des brevets

(11) EP 1 729 734 B1

(12) EUROPEAN PATENT SPECIFICATION

(45) Date of publication and mention of the grant of the patent:
**27.02.2008 Bulletin 2008/09**

(21) Application number: **05730200.2**

(22) Date of filing: **28.03.2005**

(51) Int Cl.:
***A61K 9/16*** $^{(2006.01)}$ ***A61K 31/47*** $^{(2006.01)}$

(86) International application number:
**PCT/US2005/010350**

(87) International publication number:
**WO 2005/097077 (20.10.2005 Gazette 2005/42)**

(54) **SPRAY DRIED PHARMACEUTICAL COMPOSITIONS**

SPRÜHGETROCKNETE PHARMAZEUTISCHE ZUSAMMENSETZUNGEN

COMPOSITIONS PHARMACEUTIQUES SECHEES PAR PULVERISATION

(84) Designated Contracting States:
**AT BE BG CH CY CZ DE DK EE ES FI FR GB GR HU IE IS IT LI LT LU MC NL PL PT RO SE SI SK TR**
Designated Extension States:
**HR LV**

(30) Priority: **30.03.2004 US 557571 P**

(43) Date of publication of application:
**13.12.2006 Bulletin 2006/50**

(73) Proprietor: **SMITHKLINE BEECHAM CORPORATION**
**Philadelphia, PA 19101 (US)**

(72) Inventors:
- **ANDRONIS, Vlasios, GlaxoSmithKline King of Prussia, PA 19406 (US)**
- **PAN, Rennan, GlaxoSmithKline King of Prussia, PA 19406 (US)**
- **PATEL, Kamlesh Rameshchandra, GlaxoSmithKline King of Prussia, PA 19406 (US)**

(74) Representative: **Dolton, Peter Irving Ernest et al**
**GlaxoSmithKline**
**Corporate Intellectual Property (CN9.25.1)**
**980 Great West Road**
**Brentford, Middlesex TW8 9GS (GB)**

(56) References cited:
**EP-A- 0 499 299**
**WO-A-02/094187**
**US-A1- 2002 169 181**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to novel compositions containing the $NK_3$ receptor antagonist talnetant which compositions have enhanced bioavailability. In addition, the invention relates to processes for the preparation and to uses of the compositions in therapy.

Talnetant, (S)-(-)-*N*-(α-ethylbenzyl)-3-hydroxy-2-phenylquinoline-4-carboxamide, (alternatively 3-hydroxy-2-phenyl-*N*-[(1S)-1-phenylpropyl]-4-quinolinecarboxamide), has the chemical structure (A).

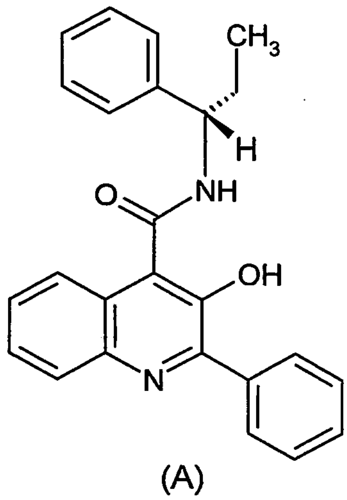

(A)

Talnetant, its preparation and its use in the treatment of pulmonary disorders, disorders of the central nervous system and neurodegenerative disorders are disclosed in published International Patent application WO 95/32948. Published International Patent applications WO 97/19927, WO 97/19928, WO 99/14196 and WO 02/094187 disclose additional therapeutic utilities for talnetant, pharmaceutically acceptable salts and processes for its preparation.

Talnetant has low aqueous solubility (approximately 0.03 mg/ml at pH 1 and 0.001 mg/ml, at pH 7.0). Typically drugs with low aqueous solubility are absorbed slowly across the walls of the gastrointestinal tract (GIT) due to poor dissolution of the solid in the GIT leading to a small diffusive driving force.

There are a number of different methods employed to improve absorption of a particular drug substance. It may be possible to develop so-called prodrugs or salts of the active agent, i.e. more soluble derivatives, by attaching a solubilizing group (*e.g.* phosphate, succinate or polyethylene glycol) to the drug, thereby taking advantage of the high solubility and dissolution rate of the derivative prodrug/salt. Alternatively, it is known to use physical formulation methods, such as use of amorphous drug or dispersion in a soluble carrier to increase the dissolution rate of the drug product and hence the absorption rate (J.H. Fincher, J. Pharm. Sci., 1968, 57, 1825 and G.L. Amidon et al., J. Pharm. Sci., 1980, 12, 1363).

A further alternative is to decrease the particle size of the drug. Decreasing the particle size increases the surface area of the drug particle, thereby increasing its dissolution rate.

A variety of processes have been developed to prepare fine particles of drug substance. Typically, dry milling techniques are used for the preparation of particulate medicaments (see E.L. Parrott, J. Pharm. Sci., 1974, 63, 813). Air jet milling and fluid energy milling (micronising) have been favoured because of the reduced risk of introducing contamination from mill materials. More recently, particles having a size of less than 1 μm have been obtained using wet milling processes. For example, published European Patent application EP-A-0 262 560 describes the use of wet milling techniques to prepare compositions containing benzoyl urea derivatives in which the average particle size is 1 μm or less. Provision of the fine particles is said to improve the absorbability from the GIT of the poorly water soluble benzoyl urea compounds thereby increasing their bioavailability. Published European Patent application EP-A-0 499 299 describes a wet milling procedure for preparing particles of a crystalline drug substance, which particles have a surface modifier adsorbed on the surface in an amount sufficient to maintain an 'effective average particle size' of less than about 400 nm.

Aqueous dispersions obtained from wet milling processes may be used directly as a therapeutic agent if prepared under conditions of appropriate hygiene, for example, by using water and other components which meet Ph Eur/USP standards. For the preparation of formulations for use in human therapy, it is preferred that the aqueous dispersion is converted to a dried powder. This is suitably carried out by spray drying the resulting aqueous dispersion, typically

collecting the product from the dryer using a cyclone separator. The resulting aqueous dispersion may a lso be spray granulated.

The objective of spray drying is to remove water from dispersions of drug particles so that the powder can be processed further to prepare capsule or tablet or other suitable oral dosage form. However, it is desirable that particles obtained from the spray dried powder are substantially the same size when dispersed in aqueous medium as the freshly-milled particles. If particles of the same size as the freshly-milled particles are obtained, it is referred to in the art (and hereinafter) as complete "recovery of particle size".

However, we have found that spray drying certain wet-milled dispersions of talnetant results in a poor recovery of particle size, i.e. a significant increase in particle size is seen when the spray dried particles are added to aqueous media.

We have discovered that spray drying wet-milled dispersions of talnetant containing suitable excipients addresses this problem, resulting in increased recovery of particle size and greatly improving bioavailability *in vivo.*

According to a first aspect, the invention provides a process for the preparation of a spray-dried composition, the composition comprising i) talnetant particles having a $D_v90$ in the range from 0.1 to 2.0 μm, ii) one or more ionic surfactant and iii) one or more soluble carrier, the process comprising a) wet milling a dispersion of the solid talnetant particles until the $D_v90$ is in the range from 0.1 to 2.0 μm, which dispersion comprises the one or more ionic surfactant and the one or m ore soluble carrier, then b) spray drying or spray granulating the resulting dispersion.

As used herein, the term $D_v90$ means that no less than 90% of the particles have a volume mean diameter of $D_v$. Similarly $D_v50$ and $D_v10$ means that not less than 50% and 10% respectively have a volume mean diameter of $D_v$.

As used herein with reference to particle size, the term "volume mean diameter" ($D_v$) means,

$$D_v = \sqrt[3]{\frac{\sum nD^3}{\sum n}}$$

where, n is the number of particles and D is the diameter of the particles, in accordance with, E. L. Parrott., "Milling", Chapter 2, The Theory and Practice of Industrial Pharmacy, Ed. Lachman et al., Third Edition, 1986, page 26.

Talnetant may be wet-milled in any suitable aqueous, non-aqueous or organic solvent (e.g. an oil). In an embodiment, talnetant is wet-milled in a water-based medium.

Suitable milling apparatus for the preparation of compositions according to the present invention include, conventional wet bead mills such as those manufactured by Nylacast (available from Nylacast Components, 200 Hastings Road, Leicester, LE5 0HL, UK), Netzsch (available from Erich NETZSCH GmbH & Co. Holding KG Gebrüder-Netzsch-Straße 19, D-95100 Selb, Germany), Drais (available from Draiswerke, Inc, 40 Whitney Road, Mahwah, NJ 07430, USA) and others. Suitably, the milling chamber of the milling apparatus is lined with or constructed from an abrasion-resistant polymer material. The milling chamber of the milling apparatus may be lined with or constructed from nylon. An example of a suitable milling chamber is described in International Patent Publication WO 02/00196.

Suitable grinding media for use in the preparation of a pharmaceutical composition according to the present invention include glass beads and ceramic beads, for example, those made from rare earth oxide materials. The diameter of said grinding media is for example within the range 0.1 mm to 3 mm, suitably within the range 0.3 mm to 0.8 mm. The density of said grinding media is for example greater than 3 $gcm^{-3}$, suitably within the range 5 to 10 $gcm^{-3}$.

Suitable spray drying and spray granulating techniques will be apparent to those skilled in the art (see for example, Gilbert S. Banker, "Modern Pharmaceutics, Drugs and the Pharmaceutical Sciences", 1996 and references cited therein) and may be effected using a spray dryer, such as the Niro SD 6.3R Spray Dryer (Niro A/S, Gladsaxevej 305, 2860 Soeborg, Denmark), the Niro Mobile Minor, the Yam ato GA-32 Spray Dryer (2-1-6 Nihonbashi Honcho, Chuo-ku, Tokyo, 103-8432, Japan) or a fluid bed granulator, such as the Glatt fluid bed granulator.

Particles prepared according to the present invention may be sized using conventional techniques known in the art, such as laser light diffraction and photon correlation spectroscopy. A suitable particle sizing apparatus is the Malvern Mastersizer 2000 (available from Malvern Instruments Ltd, Malvern, UK). A further suitable particle sizing apparatus is the Sympatec HELOS (available from Sympatec GmbH, System-Partikel-Technik, Am Pulverhaus 1, D-38678 Clausthal-Zellerfeld, Germany). The Malvern Mastersizer 2000 and the Sympatec HELOS and their operation will be familiar to the skilled person with reference to their operating manuals.

In an embodiment, the dispersion contains 5 to 50 % w/w of talnetant. In a further embodiment, the dispersion contains 15 to 30 % w/w of talnetant.

The ionic surfactant may be an anionic surfactant or a cationic surfactant. In an embodiment, the ionic surfactant is an anionic surfactant. In a further embodiment, the ionic surfactant is sodium lauryl sulfate or dioctyl sodium sulfosuccinate (docusate sodium). In a still further embodiment the ionic surfactant is sodium lauryl sulfate.

In an embodiment, the concentration of surfactant in the spray dried composition is 0.5 to 3.0% by weight of

talnetant. In an embodiment, the concentration of surfactant in the dispersion prior to spray drying is 0.05 to 5.0% by weight of dispersion, in a further embodiment 0.05 to 2.0%.

In an embodiment, the dispersion contains 0.001 to 0.1 moles of ionic surfactant per mole of talnetant. In a further embodiment, the dispersion contains 0.01 to 0.05 moles of surfactant per mole of talnetant.

In an embodiment the one or more soluble carrier is a soluble sugar. In an embodiment, the one or more soluble carrier is selected from the group consisting of mannitol, sorbitol, lactose, lactitol, xylitol, trehalose, dextrose, sucrose, maltose, fructose, maltilol, xylitol, erythritol, polydextrose, isomalt, cyclodextrin and starch. In a further embodiment, the spray dried composition comprises one or more soluble carrier selected from the group consisting of mannitol, lactose, erythritol, polydextrose, isomalt and lactitol.

In an embodiment, the concentration of the one or more soluble carrier in the spray dried composition is 10 to 75 % by weight of talnetant. The one or more soluble carrier may be added to the dispersion prior to wet milling. Alternatively, the one or more soluble carrier may be added to the wet milled dispersion before spray drying. In an embodiment, the concentration of the one or more soluble carrier in the dispersion prior to wet milling or after wet milling is 0.1 to 30% by weight of dispersion. In a further embodiment, the concentration of the soluble carrier in the dispersion prior to wet milling or after wet milling is 5 to 15% by weight of dispersion.

In an embodiment, the spray-dried composition comprises one or more anti-agglomeration agents (for example polyvinyl pyrolidone (PVP) or povidone, hydroxypropyl methyl cellulose and hydroxyethyl cellulose and hydroxypropylcellulose). In an embodiment, the concentration of the anti-aglomeration agent in the spray-dried composition is 2 to 10% by weight of talnetant. In an embodiment, the concentration of anti-aglomeration agent in the dispersion prior to spray drying is 0.1 to 10.0% by weight of dispersion, in a further embodiment 0.5 to 5.0%.

According to a second aspect, the invention provides a spray dried pharmaceutical composition comprising i) talnetant particles having a $D_v90$ in the range from 0.1 to 2.0 μm, ii) one or more ionic surfactant and iii) one or more soluble carrier.

It will be appreciated that the embodiments described for the first aspect extend to this second aspect.

The spray-dried composition and dispersion (prior to wet milling or after wet milling) may contain further suitable pharmaceutically acceptable excipients. Suitable excipients are described in the Handbook of Pharmaceutical Excipients, Pharmaceutical Press, 1986, published by The American Pharmaceutical Association and The Royal Pharmaceutical Society of Great Britain. Examples of further excipients include stablilisers to maintain the particles in suspension.

The spray dried composition may be administered to the subject without further processing, however it will generally be formulated into other dosage forms in conjunction with further pharmaceutically acceptable excipients selected with regard to the desired dosage form. These further excipients will typically be added to the spray dried composition after spray drying. Therefore according to a third aspect there is provided a dosage form comprising a composition defined in the second aspect.

It will be appreciated that the embodiments described for the first and second aspects extend to this third aspect.

In an embodiment, the dosage form is administered orally. Oral administration will typically involve swallowing so that the compound enters the GIT. Dosage forms for oral administration include solid formulations such as tablets, capsules (containing particulates, powders or non-aqueous suspension), sachets, vials, powders, granules, lozenges, reconstitutable powders and liquid preparations (such as suspensions, emulsions and elixirs).

Oral dosage forms may contain further excipients such as binding agents (for example syrup, acacia, gelatin, sorbitol, starch, PVP, HPMC, and tragacanth); fillers (for example lactose, sugar, maize-starch, calcium phosphate, sorbitol and glycine); tabletting lubricants (for example magnesium stearate); and disintegrants (for example starch, crospovidone, croscarmellose sodium, sodium starch glycollate and microcrystalline cellulose). In addition, the oral dosage form may contain preservatives, anti-oxidant, flavours, granulation binders, wetting agents and colorants.

In an embodiment, the dosage form for oral administration is a tablet. Tablets may be prepared using standard technology familiar to the formulation scientist, for example by direct compression, granulation, melt congealing and extrusion. The tablet may be coated or uncoated. The tablet may be formulated to be immediate or controlled release. Controlled release formulations include delayed-, sustained-, pulsed- or dual-release. Suitable tabletting excipients are described in the Handbook of Pharmaceutical Excipients, Pharmaceutical Press, 1986, published by The American Pharmaceutical Association and The Royal Pharmaceutical Society of Great Britain. Typical tabletting excipients include: carriers [for example microcrystalline cellulose (Avicel PH 102)], lubricating agents (for example magnesium stearate), binding agents, wetting agents, colorants, flavourings, glidants [for example Colloidal Silicon Dioxide (Cab-O-Sil M-5 P)] and disintegrants [for example crospovidone (Polyplasdone XL)].

In a further embodiment the tablet consists of the excipients in composition A of Example 2 hereinafter.

Excipients suitable for preparing liquid dosage forms include: suspending agents (for example sorbitol, syrup, methyl cellulose, gelatin, hydroxyethylcellulose, carboxymethylcellulose, aluminium stearate gel and hydrogenated edible fats); emulsifying agents (for example lecithin, sorbitan monooleate and acacia); aqueous or non-aqueous vehicles, which include edible oils (for example almond oil and fractionated coconut oil), oily esters (for example esters of glycerine and propylene glycol), ethyl alcohol, glycerine, water and normal saline; preservatives (for example methyl, propyl p-hydroxybenzoate and sorbic acid); and if desired conventional flavouring or colouring agents.

The effective dose of talnetant depends on the condition of the patient, the frequency and route of administration. A unit dose will generally contain from 20 to 1000 mg of talnetant, in an embodiment 30 to 500 mg, in a further embodiment 200 or 400 mg. The unit dose may be administered one or more times per day (for example 2, 3 or 4 times per day). The total daily dose for a 70 kg adult will normally be in the range 100 to 3000 mg. Alternatively the unit dose will contain from 2 to 20 mg of active ingredient and be administered in multiples, if desired, to give the preceding daily dose.

In an embodiment, the compositions and tablets of the invention are adapted for use in the medical or veterinarial fields. For example, such preparations may be in a pack form accompanied by written or printed instructions for use as an agent in the treatment of the conditions.

$NK_3$ receptor antagonists, including talnetant, are useful in the treatment and prevention of a wide variety of clinical diseases and conditions characterised by overstimulation of the $NK_3$ receptors. These diseases and conditions (hereinafter referred to as "diseases and conditions of the invention") include: CNS disorders such as depression (which term includes bipolar (manic) depression (including type I and type II), unipolar depression, single or recurrent major depressive episodes with or without psychotic features, catatonic features, melancholic features, atypical features (e.g. lethargy, over-eating/obesity, hypersomnia) or postpartum onset, seasonal affective disorder and dysthymia, depression-related anxiety, psychotic depression, and depressive disorders resulting from a general medical condition including, but not limited to, myocardial infarction, diabetes, miscarriage or abortion); anxiety disorders (including generalised anxiety disorder, social anxiety disorder, agitation, tension, social or emotional withdrawal in psychotic patients, panic disorder, and obsessive compulsive disorder); phobias (including agoraphobia and social phobia); psychosis and psychotic disorders (including schizophrenia, schizo-affective disorder, schizophreniform diseases, acute psychosis, alcohol psychosis, autism, delerium, mania (including acute mania), manic depressive psychosis, hallucination, endogenous psychosis, organic psychosyndrome, paranoid and delusional disorders, puerperal psychosis, and psychosis associated with neurodegenerative diseases such as Alzheimer's diease); post-traumatic stress disorder; attention deficit hyperactive disorder; cognitive impairment (e.g. the treatment of impairment of cognitive functions including attention, orientation, memory (memory disorders, amnesia, amnesic disorders and age-associated memory impairment) and language function, and including cognitive impairment as a result of stroke, Alzheimer's disease, Aids-related dementia or other dementia states, as well as other acute or sub-acute conditions that may cause cognitive decline such as delirium or depression (pseudodementia states)); convulsive disorders such as epilepsy (which includes simple partial seizures, complex partial seizures, secondary generalised seizures, generalised seizures including absence seizures, myoclonic seizures, clonic seizures, tonic seizures, tonic clonic seizures and atonic seizures); psychosexual dysfunction (including inhibited sexual desire (low libido), inhibited sexual arousal or excitement, orgasm dysfunction, inhibited female orgasm and inhibited male orgasm, hypoactive sexual desire disorder, female sexual desire disorder, and sexual dysfunction side-effects induced by treatment with antidepressants of the SSRI-class); sleep disorders (including disturbances of circadian rhythm, dyssomnia, insomnia, sleep apnea and narcolepsy); disorders of eating behaviours (including anorexia nervosa and bulimia nervosa); neurodegenerative diseases (such as alzheimer's disease, amyotropic lateral sclerosis, motor neuron disease and other motor disorders such as Parkinson's disease (including relief from locomotor deficits and/or motor disability, including slowly increasing disability in purposeful movement, tremors, bradykinesia, hyperkinesia (moderate and severe), akinesia, rigidity, disturbance of balance and co-ordination, and a disturbance of posture), dementia in Parkinson's disease, dementia in Huntington's disease, neuroleptic-induced Parkinsonism and tardive dyskinesias, neurodegeneration following stroke, cardiac arrest, pulmonary bypass, traumatic brain injury, spinal cord injury or the like, and demyelinating diseases such as multiple sclerosis and amyotrophic lateral sclerosis); withdrawal from abuse of drugs including smoking cessation or reduction in level or frequency of such activities (such as abuse of cocaine, ethanol, nicotine, benzodiazepines, alcohol, caffeine, phencyclidine and phencyclidine-like compounds, opiates such as cannabis, heroin, morphine, sedative, hypnotic, amphetamine or amphetamine-related drugs such as dextroamphetamine, methylamphetamine or a combination thereof); pain (which includes neuropathic pain (including diabetic neuropathy; sciatica; non-specific lower back pain; multiple sclerosis pain; pain associated with fibromyalgia or cancer; AIDS-related and HIV-related neuropathy; chemotherapy-induced neuropathy; neuralgia, such as post-herpetic neuralgia and trigeminal neuralgia; sympathetically maintained pain and pain resulting from physical trauma, amputation, cancer, toxins or chronic inflammatory conditions such as rheumatoid arthritis and osteoarthritis; reflex sympathetic dystrophy such as shoulder/hand syndrome), acute pain (e.g. musculoskeletal pain, post operative pain and surgical pain), inflammatory pain and chronic pain, pain associated with normally non-painful sensations such as "pins and needles" (paraesthesias and dysesthesias), increased sensitivity to touch (hyperesthesia), painful sensation following innocuous stimulation (dynamic, static or thermal allodynia), increased sensitivity to noxious stimuli (thermal, cold, mechanical hyperalgesia), continuing pain sensation after removal of the stimulation (hyperpathia) or an absence of or deficit in selective sensory pathways (hypoalgesia), pain associated with migrane, and non-cardiac chest pain); certain CNS-mediated disorders (such as emesis, irritable bowel syndrome and non-ulcer dyspepsia); and pulmonary disorders (such as asthma, chronic obstructive pulmonary disease, airway hyperreactivity and cough).

More preferred diseases or conditions (hereinafter referred to as "preferred diseases and conditions of the

invention") mediated by modulation of the $NK_3$ receptor are depression; anxiety disorders; phobias; psychosis and psychotic disorders; post-traumatic stress disorder; attention deficit hyperactive disorder; withdrawal from abuse of drugs including smoking cessation or reduction in level or frequency of such activities; irritable bowel syndrome; cognitive impairment; convulsive disorders; psychosexual dysfunction; sleep disorders; disorders of eating behaviours; neurodegenerative diseases; pain; emesis; irritable bowel syndrome; non-ulcer dyspepsia; and pulmonary disorders (such as asthma, chronic obstructive pulmonary disease, airway hyperreactivity and cough).

The following Examples illustrate the present invention.

Example 1

Preparation of spray dried compositions and particle-size recovery after dispersion in water

a) Composition 1 (according to the invention)

Sodium lauryl sulphate (0.3% w/w) and povidone (Kollidone K30) (1.7% w/w) were dissolved in purified water (68.0% w/w). Solid talnetant (20.0% w/w, $D_v90$ approximately 20 to 30 μm) was then slowly added with continuous mixing until a homogeneous suspension was obtained. The homogenous suspension was passed through a Netzsch bead mill (containing 85% by volume of yttrium-stabilised 0.3 mm zirconium oxide beads). The dispersion was re-circulated through the bead mill with continual mixing until a $D_v90$ of >0.4 μm <1.0 μm was obtained. The $D_v90$ was measured using a Malvern Mastersizer 2000. The results are shown in Table 1. Mannitol powder USP (10.0% w/w) was then added to the dispersion until a uniform suspension was obtained. This dispersion was spray-dried using a Niro Mobile Minor spray dryer (operated in accordance with the manufacturers instructions) at the following settings: two-fluid nozzle: 2 bar atomisation pressure; drying gas flowrate: 65 $m^3$/hr; suspension spray rate: 35 mL/min; inlet temperature: 150°C; outlet temperature: 60°C. A sample of the spray-dried composition was dispersed in water and the $D_v90$ was measured using a Malvern Mastersizer 2000. The results are shown in Table 1.

b) Composition 2 (according to the invention)

Dioctyl sulfosucciniate sodium (30.2 g, 0.3% w/w), povidone (Kollidone K30) (170 g, 1.7% w/w) and mannitol powder USP (1001.9 g, 10.0% w/w) were dissolved in purified water (6820 g, 68.0% w/w). Solid talnetant (2012 g, 20.0% w/w, $D_v90$ approximately 20 to 30 μm) was then slowly added with continuous mixing until a homogeneous suspension was obtained. The homogenous suspension was passed through a Netzsch bead mill as described for composition 1. The dispersion was re-circulated tbrought the bead mill with continual mixing until a $D_v90$ of >0.4 μm <1.0 μm was obtained. The $D_v90$ was measured using a Malvern Mastersizer 2000. The results are shown in Table 1. This dispersion was spray-dried using a Mobile Minor Niro spray dryer (operated in accordance with the manufacturers instructions) at the following settings: two-fluid nozzle: 2 bar atomisation pressure; suspension spray rate 35 mL/min; inlet temperature: 150°C; outlet temperature: 60°C. The spray-dried composition was dispersed in water and the $D_v90$ was measured using a Malvern Mastersizer 2000. The results are shown in Table 1.

c) Composition 3 (according to the invention)

Sodium lauryl sulfate (30 g, 0.3% w/w) was dissolved in purified water (6470.3 g, 64.7% w/w). To this stirred solution were slowly added povidone (Kollidone K30) (500.1 g, 5.0% w/w), lactose monohydrate (impalpable #312) (999.7 g, 10.0% w/w) and solid talnetant (2000.5 g, 20.0% w/w, $D_v90$ approximately 20 to 30 μm) until a homogeneous suspension was obtained. The homogenous suspension was passed through a Netzsch bead mill as described for composition 1. The dispersion was re-circulated with continual mixing until a $D_v90$ of >0.4 μm <1.0 μm was obtained. The $D_v90$ was measured using a Malvern Mastersizer 2000. The results are shown in Table 1. This dispersion was spray-dried using a Mobile Minor Niro spray dryer (operated in accordance with the manufacturers instructions) at the following settings: two-fluid nozzle: 2 bar atomisation pressure; suspension spray rate: 35 mL/min; inlet temperature: 150°C; outlet temperature: 60°C. The spray-dried composition was dispersed in water and the $D_v90$ was measured using a Malvern Mastersizer 2000. The results are shown in Table 1.

d) Composition 4 (comparative example - without ionic surfactant and soluble carrier)

Pluronic F68 (a non-ionic surfactant) (3.9% w/w) was added slowly to purified water (74.5 % w/w) followed by solid talnetant (19.6% w/w, $D_v90$ approximately 20 to 30 μm) with continuous mixing until a homogeneous suspension was obtained. The homogenous suspension was passed through a Netzsch bead mill as described for composition 1. The dispersion was re-circulated through the bead mill with continual mixing until a $D_v90$ of >0.4 μm <1.0 μm was

obtained. The $D_v90$ was measured using a Malvern Mastersizer 2000. The results are shown in Table 1. Povidone (Kollidone K30) (2.0% w/w) was then added to the dispersion until a uniform suspension was obtained. This dispersion was spray-dried using a Niro Mobile Minor spray dryer (operated in accordance with the manufacturers instructions) at the following settings: two-fluid nozzle: 2 bar atomisation pressure; drying gas flowrate: 65 $m^3$/hr; suspension spray rate: 35 mL/min; inlet temperature: 110 °C; outlet temperature: 40 °C. A sample of a spray-dried composition was dispersed in water and the $D_v90$ was measured using a Malvern Mastersizer 2000. The results are shown in Table 1.

The particle size distribution following redispersion in water for compositions 1 to 4 is shown in Table 1 (the values in parentheses are the corresponding $D_v$ values of the wet milled suspension before spray drying). The table shows that compositions 1, 2 and 3 gave virtually complete recovery of particle size after redispersion in water, whereas composition 4 gave poor recovery.

Table 1

| | Composition 1 | Composition 2 | Composition 3 | Composition 4 |
|---|---|---|---|---|
| $D_v10$ (μm) | 0.08 (0.08) | 0.08 (0.08) | 0.08 (0.07) | 0.13 (0.08) |
| $D_v50$ (μm) | 0.18 (0.19) | 0.18 (0.19) | 0.18 (0.17) | 0.55 (0.18) |
| $D_v90$ (μm) | 0.44 (0.49) | 0.64 (0.65) | 0.59 (0.41) | 2.75 (0.41) |

Example 2

Comparative study to evaluate the effect on pK parameters of oral administration of compositions of talnetant to conscious, male beagle dogs.

A catheter was placed in the cephalic vein of each of four fasted male beagle dogs. Each dog was administered half a Tablet A (the composition of which is shown in Table 2 - the dosage form of the invention). Blood samples were collected via the catheter prior to dosing and at the following times after dosing: 15, 30, 45, 60, 90, 120, 180, 240, 300, 360, 480, 600 and 1440 minutes. Plasma was prepared and stored frozen for analysis. Plasma concentrations of talnetant (limit of quantitation = 10 ng/mL) were quantified by an LC/MS/MS method. Non-compartmental analysis was used for pharmacokinetic analysis of plasma concentration versus actual sampling time data. The dogs were fed at 6 hours post-dose (following the collection of the 6-hour blood sample) and food was removed one hour later.

After a wash-out period of one week, the procedure was repeated on the same four dogs with Tablet B. After a further one week wash-out period, the procedure was repeated with Tablet C, and so on with Tablet D (Tablets B, C and D are comparative examples).

Table 2

| Tablet | Constituents (mg/tablet) | Dose (mg/kg) |
|---|---|---|
| A | Talnetant[a] (100)<br>Sodium Lauryl Sulfate[a] (1.5)<br>Povidone[a] (8.5)<br>Mannitol[a] (50)<br>Avicel PH 200[b] (169.4)<br>Crospovidone[b] (38.70)<br>Colloidal Silicon Dioxide[b] (4)<br>Magnesium Stearate[b] (2.9)<br>Opadry yellow[b] (11.25) | 5.46 ± 0.60<br>(n=3) |
| B | Talnetant[a] (100)<br>Poloxamer[a] 127 (8.5)<br>Povidone[a] (5)<br>Natrosol 250SL[a] (10)<br>Avicel PH 200[b] (205.9)<br>Crospovidone[b] (38.70)<br>Colloidal Silicon Dioxide[b] (4)<br>Magnesium Stearate[b] (2.9) | 5.09 ± 0.66<br>(n=4) |

(continued)

| Tablet | Constituents (mg/tablet) | Dose (mg/kg) |
|---|---|---|
| | Opadry yellow[b] (11.25) | |
| C | Talnetant[a] (100)<br>Poloxamer 188[a] (20.03)<br>Povidone[a] (10.01)<br>Avicel PH 200[b] (199.36)<br>Crospovidone[b] (38.70)<br>Colloidal Silicon Dioxide[b] (4)<br>Magnesium Stearate[b] (2.9)<br>Opadry yellow[b] (11.25) | 5.43 ± 0.76<br>(n=4) |
| D | Talnetant[a] (100)<br>Poloxamer 188[a] (10)<br>Povidone[a] (10)<br>Avicel PH 200[b] (209.4)<br>Crospovidone[b] (38.70)<br>Colloidal Silicon Dioxide[b] (4)<br>Magnesium Stearate[b] (2.9)<br>Opadry yellow[b] (11.25) | 5.49 ± 0.68<br>(n=4) |

The spray dried compositions used in tablets A, B, C and D were prepared by combining ingredients marked with superscript "a" in similar manner to the methods described in Example 1. Each dispersion was wet-milled to give a $D_v90$ of 0.5 μm. After spray drying, the compositions were tabletted using the ingredients marked with superscript "b" using standard tabletting technology familiar to the skilled person.

The results of the study are shown in Table 3. The results show that administration of Tablet A (the composition of the invention) lead to significant improvement in bioavailability compared with the comparative examples (Tablets B, C and D). In addition the results show that the values for Tablet A were the most consistent among the subjects.

The average relative bioavailability for the three formulations against Tablet C was calculated by determining the relative bioavailibility for each animal then calculating the average and standard deviation of the individual relative bioavailability values. Overall, the average relative bioavailability of Table A was 1.96 ± 0.34, and the individual values were 1.81, 2.35 and 1.72.

$C_{max}$ is the maximum plasma concentration of talnetant achieved. $T_{max}$ is the time after administration at which the maximum concentration was achieved. AUC is the Area Under the Curve of a plot of plasma concentration against time. AUC(0-t) refers to the area from time zero to the last quantifiable concentration at time t. AUC (0-360) refers to the area from time zero to t = 360 minutes.

Table 3

| Tablet | $C_{max}$ (μg/mL) | $T_{max}$ (min) | AUC (0-360) (min. μg/mL) | AUC (0-t) (min. μg/mL) | Relative Oral Bioavailability $\frac{AUC(0\text{-}360)}{AUC(0\text{-}360)_{Tablet\ C}}$ |
|---|---|---|---|---|---|
| A | 4.04 ± 0.287 | 521.0 ± 68.4 | 742.1 ± 55.0 | 3306.0 ± 117.2 | 1.96 ± 0.34 |
| B | 2.20 ± 0.796 | 480.0 ± 0.0 | 345.7 ± 139.2 | 1373.4 ± 730.3 | 1.03 ± 0.23 |
| C | 2.15 ± 0.669 | 278.3 ± 307.5 | 336.8 ± 121.2 | 1654.0 ± 1354.8 | 1.00 ± 0.00 |
| D | 1.94 ± 0.850 | 419.8 ± 206.3 | 305.3 ± 109.8 | 1391.1 ± 395.7 | 0.94 ± 0.27 |

## Claims

1. A process for the preparation of a spray-dried composition, the composition comprising i) talnetant particle, wherein no less than 90% of the particles have a volume mean diameter ($D_v90$) in the range from 0.1 to 2.0 μm, ii) one or

more ionic surfactant and iii) one or more soluble carrier, the process comprising a) wet milling a dispersion of the solid talnetant particles until the $D_v90$ is in the range from 0.1 to 2.0 μm, which dispersion comprises the one or more ionic surfactant and the one or more soluble carrier, then b) spray drying or spray granulating the resulting dispersion.

**2.** A process according to claim 1 wherein the dispersion is wet-milled in a water-based medium.

**3.** A process according to any preceding claim wherein the dispersion contains 5 to 50 % w/w of talnetant.

**4.** A process according to any preceding claim wherein the dispersion contains 15 to 30 % w/w of talnetant.

**5.** A process according to any preceding claim wherein the ionic surfactant is an anionic surfactant.

**6.** A process according to any preceding claim wherein the ionic surfactant is sodium lauryl sulfate or dioctyl sodium sulfosuccinate.

**7.** A process according to any preceding claim wherein the ionic surfactant is sodium lauryl sulfate.

**8.** A process according to any preceding claim wherein the concentration of surfactant in the spray dried composition is 0.5 to 3.0% by weight of talnetant.

**9.** A process according to any preceding claim wherein the concentration of surfactant in the dispersion prior to spray drying is 0.05 to 5.0% by weight of dispersion.

**10.** A process according to any preceding claim wherein the dispersion contains 0.001 to 0.1 moles of ionic surfactant per mole of talnetant.

**11.** A process according to any preceding claim wherein the one or more soluble carrier is a soluble sugar.

**12.** A process according to any preceding claim wherein the one or more soluble carrier is selected from the group consisting of mannitol, sorbitol, lactose, lactitol, xylitol, trehalose, dextrose, sucrose, maltose, fructose, maltilol, xylitol, erythritol, polydextrose, isomalt, cyclodextrin and starch.

**13.** A process according to any preceding claim wherein the spray dried composition comprises one or more soluble carrier selected from the group consisting of mannitol, lactose, erythritol, polydextrose, isomalt and lactitol.

**14.** A process according to any preceding claim wherein the concentration of the one or more soluble carrier in the spray dried composition is 10 to 75 % by weight of talnetant.

**15.** A process according to any preceding claim wherein the concentration of the one or more soluble carrier in the dispersion prior to wet milling or after wet milling is 0.1 to 30% by weight of dispersion.

**16.** A process according to any preceding claim wherein the spray-dried composition comprises one or more anti-agglomeration agents.

**17.** A process according to any preceding claim wherein the concentration of the anti-aglomeration agent in the spray-dried composition is 2 to 10% by weight of talnetant.

**18.** A process according to any preceding claim wherein the concentration of anti-aglomeration agent in the dispersion prior to spray drying is 0.1 to 10.0% by weight of dispersion.

**19.** A spray dried pharmaceutical composition comprising i) talnetant particles , wherein no less than 90% of the particles have a volume mean diameter ($D_v90$) in the range from 0.1 to 2.0 μm, ii) one or more ionic surfactant and iii) one or more soluble carrier.

**20.** A pharmaceutical composition according to claim 19 wherein the ionic surfactant is an anionic surfactant.

**21.** A pharmaceutical composition according to claim 19 or 20 wherein the ionic surfactant is sodium lauryl sulfate or

dioctyl sodium sulfosuccinate.

**22.** A pharmaceutical composition according to any one of claims 19 to 21 wherein the ionic surfactant is sodium lauryl sulfate.

**23.** A pharmaceutical composition according to any one of claims 19 to 22 wherein the concentration of surfactant in the spray dried composition is 0.5 to 3.0% by weight of talnetant.

**24.** A pharmaceutical composition according to any one of claims 19 to 23 wherein the one or more soluble carrier is a soluble sugar.

**25.** A pharmaceutical composition according to any one of claims 19 to 24 wherein the one or more soluble carrier is selected from the group consisting of mannitol, sorbitol, lactose, lactitol, xylitol, trehalose, dextrose, sucrose, maltose, fructose, maltilol, xylitol, erythritol, polydextrose, isomalt, cyclodextrin and starch.

**26.** A pharmaceutical composition according to any one of claims 19 to 25 wherein the spray dried composition comprises one or more soluble carrier selected from the group consisting of mannitol, lactose, erythritol, polydextrose, isomalt and lactitol.

**27.** A pharmaceutical composition according to any one of claims 19 to 26 wherein the concentration of the one or more soluble carrier in the spray dried composition is 10 to 75 % by weight of talnetant.

**28.** A pharmaceutical composition according to any one of claims 19 to 27 wherein the spray-dried composition comprises one or more anti-agglomeration agents.

**29.** A pharmaceutical composition according to any one of claims 19 to 28 wherein the concentration of the anti-aglomeration agent in the spray-dried composition is 2 to 10% by weight of talnetant.

**30.** A dosage form comprising a composition defined in any one of claims 19 to 29.

**31.** A dosage form according to claim 30 administered orally.

**32.** A dosage form according to claim 31 administered as a tablet.

**Patentansprüche**

**1.** Verfahren zur Herstellung einer sprühgetrockneten Zusammensetzung, wobei die Zusammensetzung i) Talnetant-Teilchen, wobei nicht weniger als 90% der Teilchen einen volumengemittelten Durchmesser ($D_v90$) im Bereich von 0,1 bis 2,0 μm haben, ii) ein oder mehrere ionische oberflächenaktive Mittel und iii) einen oder mehrere lösliche Träger umfasst, wobei das Verfahren a) Nassmahlen einer Dispersion der festen Talnetant-Teilchen bis der $D_v90$ im Bereich von 0,1 bis 2,0 μm liegt, wobei die Dispersion das eine oder die mehreren ionischen oberflächenaktiven Mittel und den einen oder die mehreren löslichen Träger umfasst, dann b) Sprühtrocknen oder Sprühgranulieren der entstandenen Dispersion umfasst.

**2.** Verfahren nach Anspruch 1, wobei die Dispersion in einem Medium auf Wasserbasis nassgemahlen wird.

**3.** Verfahren nach einem der vorangehenden Ansprüche, wobei die Dispersion 5 bis 50 Gew.-% Talnetant enthält.

**4.** Verfahren nach einem der vorangehenden Ansprüche, wobei die Dispersion 15 bis 30 Gew.-% Talnetant enthält.

**5.** Verfahren nach einem der vorangehenden Ansprüche, wobei das ionische oberflächenaktive Mittel ein anionisches oberflächenaktives Mittel ist.

**6.** Verfahren nach einem der vorangehenden Ansprüche, wobei das ionische oberflächenaktive Mittel Natriumlaurylsulfat oder Dioctylnatriumsulfosuccinat ist.

**7.** Verfahren nach einem der vorangehenden Ansprüche, wobei das ionische oberflächenaktive Mittel Natriumlaurylsulfat ist.

**8.** Verfahren nach einem der vorangehenden Ansprüche, wobei die Konzentration des oberflächenaktiven Mittels in der sprühgetrockneten Zusammensetzung 0,5 bis 3,0 Gew.-% des Talnetants ist.

**9.** Verfahren nach einem der vorangehenden Ansprüche, wobei die Konzentration des oberflächenaktiven Mittels in der Dispersion vor der Sprühtrocknung 0,05 bis 5,0 Gew.-% der Dispersion ist.

**10.** Verfahren nach einem der vorangehenden Ansprüche, wobei die Dispersion 0,001 bis 0,1 Mol des ionischen oberflächenaktiven Mittels pro Mol Talnetant enthält.

**11.** Verfahren nach einem der vorangehenden Ansprüche, wobei der eine oder die mehreren löslichen Träger ein löslicher Zucker ist.

**12.** Verfahren nach einem der vorangehenden Ansprüche, wobei der eine oder die mehreren löslichen Träger aus Mannit, Sorbit, Lactose, Lactit, Xylit, Trehalose, Dextrose, Sucrose, Maltose, Fructose, Maltit, Xylit, Erythrit, Polydextrose, Isomalt, Cyclodextrin und Stärke ausgewählt sind.

**13.** Verfahren nach einem der vorangehenden Ansprüche, wobei die sprühgetrocknete Zusammensetzung einen oder mehrere lösliche Träger, ausgewählt aus Mannit, Lactose, Erythrit, Polydextrose, Isomalt und Lactit, umfasst.

**14.** Verfahren nach einem der vorangehenden Ansprüche, wobei die Konzentration des einen oder der mehreren löslichen Träger in der sprühgetrockneten Zusammensetzung 10 bis 75 Gew.-% des Talnetants ist.

**15.** Verfahren nach einem der vorangehenden Ansprüche, wobei die Konzentration des einen oder der mehreren löslichen Träger in der Dispersion vor dem Nassmahlen oder nach dem Nassmahlen 0,1 bis 30 Gew.-% der Dispersion ist.

**16.** Verfahren nach einem der vorangehenden Ansprüche, wobei die sprühgetrocknete Zusammensetzung ein oder mehrere Anti-Agglomerationsmittel umfasst.

**17.** Verfahren nach einem der vorangehenden Ansprüche, wobei die Konzentration des Anti-Agglomerationsmittels in der sprühgetrockneten Zusammensetzung 2 bis 10 Gew.-% des Talnetants ist.

**18.** Verfahren nach einem der vorangehenden Ansprüche, wobei die Konzentration des Anti-Agglomerationsmittels in der Dispersion vor dem Sprühtrocknen 0,1 bis 10,0 Gew.-% der Dispersion ist.

**19.** Sprühgetrocknetes Arzneimittel, umfassend i) Talnetant-Teilchen, wobei nicht weniger als 90% der Teilchen einen volumengemittelten Durchmesser ($D_v90$) im Bereich von 0,1 bis 2,0 $\mu$m haben, ii) ein oder mehrere ionische oberflächenaktive Mittel und iii) einen oder mehrere lösliche Träger.

**20.** Arzneimittel nach Anspruch 19, wobei das ionische oberflächenaktive Mittel ein anionisches oberflächenaktives Mittel ist.

**21.** Arzneimittel nach Anspruch 19 oder 20, wobei das ionische oberflächenaktive Mittel Natriumlaurylsulfat oder Dioctylnatriumsulfosuccinat ist.

**22.** Arzneimittel nach einem der Ansprüche 19 bis 21, wobei das ionische oberflächenaktive Mittel Natriumlaurylsulfat ist.

**23.** Arzneimittel nach einem der Ansprüche 19 bis 22, wobei die Konzentration des oberflächenaktiven Mittels in der sprühgetrockneten Zusammensetzung 0,5 bis 3,0 Gew.-% des Talnetants ist.

**24.** Arzneimittel nach einem der Ansprüche 19 bis 23, wobei der eine oder die mehreren löslichen Träger ein löslicher Zucker ist.

**25.** Arzneimittel nach einem der Ansprüche 19 bis 24, wobei der eine oder die mehreren löslichen Träger aus Mannit, Sorbit, Lactose, Lactit, Xylit, Trehalose, Dextrose, Sucrose, Maltose, Fructose, Maltit, Xylit, Erythrit, Polydextrose, Isomalt, Cyclodextrin und Stärke ausgewählt sind.

26. Arzneimittel nach einem der Ansprüche 19 bis 25, wobei das sprühgetrocknete Arzneimittel einen oder mehrere lösliche Träger, ausgewählt aus Mannit, Lactose, Erythrit, Polydextrose, Isomalt und Lactit, umfasst.

27. Arzneimittel nach einem der Ansprüche 19 bis 26, wobei die Konzentration des einen oder der mehreren löslichen Träger in dem sprühgetrockneten Arzneimittel 10 bis 75 Gew.-% des Talnetants ist.

28. Arzneimittel nach einem der Ansprüche 19 bis 27, wobei das sprühgetrocknete Arzneimittel ein oder mehrere Anti-Agglomerationsmittel umfasst.

29. Arzneimittel nach einem der Ansprüche 19 bis 28, wobei die Konzentration des Anti-Agglomerationsmittels in dem sprühgetrockneten Arzneimittel 2 bis 10 Gew.-% des Talnetants ist.

30. Darreichungsform, umfassend ein Arzneimittel nach einem der Ansprüche 19 bis 29.

31. Darreichungsform nach Anspruch 30, oral verabreicht.

32. Darreichungsform nach Anspruch 31, verabreicht als Tablette.

## Revendications

1. Procédé pour la préparation d'une composition séchée par atomisation, composition comprenant i) des particules de talnétant, une proportion inférieure à 90 % des particules ayant un diamètre moyen en volume ($D_v90$) de 0,1 à 2,0 μm, ii) un ou plusieurs agents tensioactifs ioniques et iii) un ou plusieurs supports solubles, procédé comprenant a) le broyage par voie humide d'une dispersion des particules de talnétant solides jusqu'à ce que la $D_v90$ soit de 0,1 à 2,0 μm, dispersion qui comprend le ou les agents tensioactifs ioniques et le ou les supports solubles, puis b) le séchage par atomisation ou la granulation par atomisation de la dispersion résultante.

2. Procédé suivant la revendication 1, dans lequel la dispersion est broyée par voie humide dans un milieu à base d'eau.

3. Procédé suivant l'une quelconque des revendications précédentes, dans lequel la dispersion contient 5 à 50 % en poids/poids de talnétant.

4. Procédé suivant l'une quelconque des revendications précédentes, dans lequel la dispersion contient 15 à 30 % en poids/poids de talnétant.

5. Procédé suivant l'une quelconque des revendications précédentes, dans lequel l'agent tensioactif ionique est un agent tensioactif anionique.

6. Procédé suivant l'une quelconque des revendications précédentes, dans lequel l'agent tensioactif ionique est le laurylsulfate de sodium ou le dioctylsulfosuccinate de sodium.

7. Procédé suivant l'une quelconque des revendications précédentes, dans lequel l'agent tensioactif ionique est le laurylsulfate de sodium.

8. Procédé suivant l'une quelconque des revendications précédentes, dans lequel la concentration de l'agent tensioactif dans la composition séchée par atomisation est de 0,5 à 3,0 % en poids du talnétant.

9. Procédé suivant l'une quelconque des revendications précédentes, dans lequel la concentration d'agent tensioactif dans la dispersion avant le séchage par atomisation est de 0,05 à 5,0 % en poids de la dispersion.

10. Procédé suivant l'une quelconque des revendications précédentes, dans lequel la dispersion contient 0,001 à 0,1 mole d'agent tensioactif par mole de talnétant.

11. Procédé suivant l'une quelconque des revendications précédentes, dans lequel le ou les supports solubles consistent en un sucre soluble.

12. Procédé suivant l'une quelconque des revendications précédentes, dans lequel le ou les supports solubles sont

choisis dans le groupe consistant en mannitol, sorbitol, lactose, lactitol, xylitol, tréhalose, dextrose, saccharose, maltose, fructose, maltitol, xylitol, érythritol, polydextrose, isomalt, cyclodextrine et amidon.

13. Procédé suivant l'une quelconque des revendications précédentes, dans lequel la composition séchée par atomisation comprend un ou plusieurs supports solubles choisis dans le groupe consistant en mannitol, lactose, érythritol, polydextrose, isomalt et lactitol.

14. Procédé suivant l'une quelconque des revendications précédentes, dans lequel la concentration du ou des supports solubles dans la composition séchée par atomisation est de 10 à 75 % en poids de talnétant.

15. Procédé suivant l'une quelconque des revendications précédentes, dans lequel la concentration du ou des supports solubles dans la dispersion avant le broyage par voie humide ou après le broyage par voie humide est de 0,1 à 30 % en poids de la dispersion.

16. Procédé suivant l'une quelconque des revendications précédentes, dans lequel la composition séchée par atomisation comprend un ou plusieurs agents anti-agglomération.

17. Procédé suivant l'une quelconque des revendications précédentes, dans lequel la concentration de l'agent anti-agglomération de la composition séchée par atomisation est de 2 à 10 % du talnétant.

18. Procédé suivant l'une quelconque des revendications précédentes, dans lequel la concentration d'agent anti-agglomération dans la dispersion avant le séchage par atomisation est de 0,1 à 10,0 % en poids de la dispersion.

19. Composition pharmaceutique séchée par atomisation, comprenant i) des particules de talnétant, une proportion inférieure à 90 % des particules ayant un diamètre moyen en volume ($D_v90$) de 0,1 à 2,0 $\mu$m, ii) un ou plusieurs agents tensioactifs ioniques et iii) un ou plusieurs supports solubles.

20. Composition pharmaceutique suivant la revendication 19, dans laquelle l'agent tensioactif ionique est un agent tensioactif anionique.

21. Composition pharmaceutique suivant la revendication 19 ou 20, dans laquelle l'agent tensioactif ionique est le laurylsulfate de sodium ou le dioctylsulfosuccinate de sodium.

22. Composition pharmaceutique suivant l'une quelconque des revendications 19 à 21, dans laquelle l'agent tensioactif ionique est le laurylsulfate de sodium.

23. Composition pharmaceutique suivant l'une quelconque des revendications 19 à 22, dans laquelle la concentration de l'agent tensioactif dans la composition séchée par atomisation est de 0,05 à 3,0 % en poids du talnétant.

24. Composition pharmaceutique suivant l'une quelconque des revendications 19 à 23, dans laquelle le ou les supports solubles consistent en un sucre soluble.

25. Composition pharmaceutique suivant l'une quelconque des revendications 19 à 24, dans laquelle le ou les supports solubles sont choisis dans le groupe consistant en mannitol, sorbitol, lactose, lactitol, xylitol, tréhalose, dextrose, saccharose, maltose, fructose, maltitol, xylitol, érythritol, polydextrose, isomalt, cyclodextrine et amidon.

26. Composition pharmaceutique suivant l'une quelconque des revendications 19 à 25, ladite composition séchée par atomisation comprenant un ou plusieurs supports solubles choisis dans le groupe consistant en mannitol, lactose, érythritol, polydextrose, isomalt et lactitol.

27. Composition pharmaceutique suivant l'une quelconque des revendications 19 à 26, dans laquelle la concentration du ou des supports solubles dans la composition séchée par atomisation est de 10 à 75 % en poids du talnétant.

28. Composition pharmaceutique suivant l'une quelconque des revendications 19 à 27, ladite composition séchée par atomisation comprenant un ou plusieurs agents anti-agglomération.

29. Composition pharmaceutique suivant l'une quelconque des revendications 19 à 28, dans laquelle la concentration de l'agent anti-agglomération de la composition séchée par atomisation est de 2 à 10 % en poids du talnétant.

**30.** Forme posologique comprenant une composition définie dans l'une quelconque des revendications 19 à 29.

**31.** Forme posologique suivant la revendication 30, administrée par voie orale.

**32.** Forme posologique suivant la revendication 31, administrée sous forme d'un comprimé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9532948 A
- WO 9719927 A
- WO 9719928 A
- WO 9914196 A
- WO 02094187 A
- EP 0262560 A
- EP 0499299 A
- WO 0200196 A


### Non-patent literature cited in the description


- **J.H. FINCHER.** *J. Pharm. Sci.,* 1968, vol. 57, 1825
- **G.L. AMIDON et al.** *J. Pharm. Sci.,* 1980, vol. 12, 1363
- **E.L. PARROTT.** *J. Pharm. Sci.,* 1974, vol. 63, 813
- Milling. **E. L. PARROTT. et al.** The Theory and Practice of Industrial Pharmacy. 1986, 26
- Handbook of Pharmaceutical Excipients. Pharmaceutical Press, 1986